# EUROPEAN PATENT SPECIFICATION

(11) **EP 2 259 781 B1**
(45) Date of publication and mention of the grant of the patent: **07.05.2014**
(21) Application number: 08709274.8
(22) Date of filing: 03.03.2008
(51) Int. Cl.: A61K 9/70, A61K 31/196

(54) **PROCESS FOR PREPARING A PHARMACEUTICAL COMPOSITION WITH ANTI-INFLAMMATORY AND ANALGESIC ACTIVITY FOR ADMINISTRATION VIA A PATCH FOR EXTERNAL USE, AND COMPOSITION THUS OBTAINED**
VERFAHREN ZUR HERSTELLUNG EINER PHARMAZEUTISCHEN ZUSAMMENSETZUNG MIT ENTZÜNDUNGSHEMMENDER UND ANALGETISCHER WIRKUNG ZUR VERABREICHUNG ÜBER EIN PFLASTER ZUR TOPISCHEN ANWENDUNG UND DAMIT ERHALTENE ZUSAMMENSETZUNG
PROCÉDÉ DE PRÉPARATION D UNE COMPOSITION PHARMACEUTIQUE AYANT UNE ACTIVITÉ ANTI-INFLAMMATOIRE ET ANALGÉSIQUE DESTINÉE À ÊTRE ADMINISTRÉE VIA UN TIMBRE CUTANÉ POUR UN USAGE EXTERNE, ET COMPOSITION AINSI OBTENUE

(43) Date of publication of application: 15.12.2010
(73) Proprietor: ALTERGON S.A., CH-6901 Lugano (CH)
(72) Inventor: TROIANO, Angelo, CH-6987 Caslano (CH); ZOPPETTI, Giorgio, I-20155 Milano (IT)
(74) Representative: Gerli, Paolo
(86) International application number: PCT/EP2008/052547
(87) International publication number: WO 2009/109214

(56) References cited:
- EP-A- 0 621 263
- EP-A- 1 046 395
- MINGHETTI PAOLA ET AL: "Ex vivo study of transdermal permeation of four diclofenac salts from different vehicles." JOURNAL OF PHARMACEUTICAL SCIENCES APR 2007, vol. 96, no. 4, April 2007 (2007-04), pages 814-823, XP002503203 ISSN: 0022-3549
- DATABASE MEDLINE [Online] US NATIONAL LIBRARY OF MEDICINE (NLM), BETHESDA, MD, US 1993 FINI A ET AL: 'Diclofenac/N-(2-hydroxyethyl)pyrrolidine: a new salt for an old drug.' Database accession no. NLM8112202 & FINI A ET AL: "Diclofenac/N-(2-hydroxyethyl)pyrrolidine: a new salt for an old drug.", DRUGS UNDER EXPERIMENTAL AND CLINICAL RESEARCH 1993, vol. 19, no. 3, 1993, pages 81-88, ISSN: 0378-6501

## Description

The present invention relates to a process for preparing a pharmaceutical composition with anti-inflammatory and analgesic activity for administration via a patch for external use.

EP0621263, in the joint name of Teikoku and the present applicant Altergon, describes a formulation suitable for administration in the form of an anti-inflammatory and analgesic patch for external transdermal use, based on a salt of diclofenac, i.e. 2[(2,6-dichlorophenyl)amino]benzene-acetic acid with a cyclic organic base, having the general formula 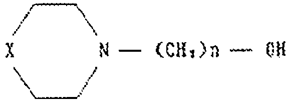 in which X is a group of formula -(CH₂)m- where m is the integer 0 or 1, and n is 2, and also comprising a pH adjuster and pharmaceutically acceptable components such as thickeners, humectants, preservatives and a cross-linking agent.

The diclofenac salt with the hydroxyethylpyrrolidine base is identified hereinafter by the abbreviation DHEP, while the salt with hydroxyethylpiperidine is identified by the abbreviation DHEPP.

The Teikoku/Altergon patent demonstrates experimentally that the patch in question shows high activity both in terms of increased transdermal permeation of the active principle and improved inhibition of oedema.

EP0621263 also describes a preparative process for said formulation, characterized by adding, always in an aqueous solution, the entire quantity of DHEP salt to the mixture of components as inferred from the descriptive examples of said patent.

In this respect, DHEP is characterized by a high solubility in water, up to about 40%, being 20 times greater than that of diclofenac sodium salt.

Regarding the remainder of the pharmaceutically acceptable components, EP0621263 involves the use, among others, of a compound selected from glycerol, propylene glycol, polyethylene glycol, 1,2-butanediol and a D-sorbitol solution as humectants in the final preparation.

The only example in EP0621263 that concerns the use of propylene glycol as a humectant is example 3, in which 10 parts % wt/wt of propylene glycol are added to the mixture after the entire quantity of DHEP, i.e. 0.65% wt/wt dissolved in water was already added to the mixture, in line with the aforestated general criteria.

An object of the present invention is to provide a process for preparing the formulation in EP0621263 which proves particularly advantageous from the industrial production viewpoint, where high quantities of mixture components are involved for which a stability of the solution over time is required. In this respect, although a low-volume solution can be quickly processed, at the industrial production stage the treatment of larger volumes requires long solubilization times, and therefore waiting time, prior to final production.

A further object of the present invention is to avoid for example component precipitation phenomena, particularly of the active principle, which would result in its incomplete solubilization, and consequent dishomogeneity of the final product, to hence ensure a sufficient time stability of the solution to be processed, independently of the quantities involved.

In accordance with the present invention, it has now been surprisingly found that these objects are advantageously attained by a process for preparing a pharmaceutical composition with anti-inflammatory and analgesic activity for administration via a patch for external use, its components including as active principle a salt of diclofenac, 2[(2,6-dichlorophenyl)amino]benzene-acetic acid, with a cyclic organic base chosen from hydroxyethylpyrrolidine or hydroxyethylpiperidine characterized in that said active principle is added to a mixture of one or more of said components, in the form of a solution in two solvents, namely water and propylene glycol, in a ratio of about 1:1 parts by weight.

The characteristics and advantages of the process of the present invention will be illustrated in greater detail in the following description.

The following examples of the present invention are given by way of non-limiting illustration.

Comparative example 1 is given for comparison purposes as an example of the prior art, in accordance with the process described in example 1 of the aforementioned EP0621263.

Comparative example 2 is given for comparison purposes as a further example of the prior art, in accordance with the process described in example 3 of the aforementioned EP0621263.

Example 1 relates instead to the process of the present invention.

### Comparative example 1

14 kg of gelatin and 14 kg of polyvinylpyrrolidone are added to 210 kg of purified water and dissolved by heating to 60°C.

140 kg of D-sorbitol solution, 35 kg of kaolin, 3.5 kg of titanium dioxide, 0.7 kg of methylparaben, 0.35 kg of propylparaben and 2.1 kg of tartaric acid are added to the resulting solution, and the mixture is sufficiently mixed.

A dispersion composed of 28 kg of sodium polyacrylate, 21 kg of sodium CMC and 5.6 kg of aluminium hydroxide, dispersed in 140 kg of 1,3-butylene glycol, are added. The dispersion is added in several portions, with mixing after each addition.

Finally a solution of 9.1 kg of DHEP, dissolved in the remaining 76.65 kg of purified water, is added and the mixture is mixed until it becomes homogeneous. The mixture thus obtained is spread over a non-woven fabric at 1000g/m².

A plastic film is placed over the coated fabric and the combination is cut into pieces of the required size to obtain a patch as described in EP0621263. The final preparation has a pH of 7.9.

### Comparative example 2

14 kg of gelatin and 21 kg of polyvinyl alcohol are added to 210 kg of purified water and dissolved by heating to 60°C.

210 kg of D-sorbitol solution, 35 kg of kaolin and 0.7 kg of propylparaben are added to the resulting solution, and the mixture is sufficiently mixed.

A solution of 4.55 kg of DHEP, dissolved in the remaining 80.85 kg of purified water, is then added and further mixing is carried out.

Finally a dispersion composed of 28 kg of sodium polyacrylate, 17.5 kg of sodium CMC and 8.4 kg of aluminium acetate dispersed in 70 kg propylene glycol are added and the mixture is mixed until it becomes homogeneous.

The mixture thus obtained is spread over a non-woven fabric at 1000 g/m².

A plastic film is placed over the coated fabric and the combination is cut into pieces of the required size to obtain a patch as described in EP0621263. The final preparation has a pH of 8.5.

### Example 1

Solutions or dispersions are prepared as described in the following steps:
*Step A*: 14 kg of gelatin and 14 kg of polyvinylpyrrolidone are added to about 112 kg of purified water, dissolved by heating to 50-65°C.
*Step B*: 21 kg of kaolin are dispersed in 120 kg of D-sorbitol solution.
*Step C*: 3.5 kg of titanium dioxide are dispersed in 10 kg of purified water.
*Step D*: 0.86 kg of EDTA are dissolved in 5 kg of purified water, heating to promote dissolution if necessary.
*Step E*: 0.7 kg of methylparaben and 0.35 kg of propylparaben are dissolved in 10.5 kg of propylene glycol.
*Step F*: 3.5 kg of tartaric acid are dissolved in 5 kg of water.
*Step G*: 21 kg of sodium CMC, 28 kg of sodium polyacrylate and 2.1 kg of aluminium glycinate are dispersed in 70 kg of butylene glycol.
*Step H*: 10 5 kg of propylene glycol are added to 10.5 kg of water and 9.1 kg of DHEP are dissolved in the mixture thus obtained, heating to 30-50°C.

The solutions/dispersions obtained in the aforesaid steps are poured into the mixer in the order described in the following sequence:
- product of Step A
- 160 kg of D-sorbitol solution
- product of Step B
- 40 kg of purified water
- product of Step C
- product of Step D, rinsing the container with 15 kg of purified water
- product of Step E after adding 1.4 kg of polysorbate 80
- 0.14 kg of fragrance
- product of Step F
- the mixer is started, said product of Step G is added and mixed for 2 minutes
- add said product of Step H, rinsing the container with about 10.25 kg of purified water then mix for a further 5 minutes 30 seconds in total until the mixture is homogeneous.

The mixture thus obtained is spread over a non-woven fabric at 1000 g/m². A protective plastic film is applied to the coated fabric and the combination is cut into pieces of the required size to obtain the patch of the invention.

By comparing the three aforegiven examples, it can be seen that in the prior art all the DHEP employed is added to the mixture of the required composition in the form of a solution in water alone, while in the case of the invention process, all the DHEP employed is added to the mixture of the required composition in the form of a solution in water and propylene glycol in a ratio of about 1:1 parts by weight, as described in example 1, step H.

Comparative example 2 shows that in the prior art propylene glycol is not used as a co-solvent with water for DHEP, but rather for obtaining a dispersion composed of sodium polyacrylate, sodium CMC and aluminium acetate, with the function of humectant in the final composition.

By experimentation it was found that the process of the present invention enables to avoid precipitation phenomena, which would otherwise lead to incomplete solubilization of the mixture, thus ensuring a sufficient time stability of the solution to be processed, independently of the quantities involved.

Since the process is hence suitable for handling large amounts of mixture based on the industrial scale preparation requirements of the composition in question, the objectives of the present invention are thus achieved.

## Claims

1. Process for preparing a pharmaceutical composition with anti-inflammatory and analgesic activity for administration via a patch for external use, its components including as active principle a salt of diclofenac, 2[(2,6-dichlorophenyl)amino]benzene-acetic acid, with a cyclic organic base chosen from hydroxyethylpyrrolidine or hydroxyethylpiperidine, **characterized in that** said active principle is added to a mixture of one or more of said components, in the form of a solution in two solvents, namely water and propylene glycol, in a ratio of 1:1 parts by weight.

2. Process according to claim 1, **characterized by** separately preparing solutions or dispersions of said components as described in the following steps:
*Step A*: gelatin and polyvinylpyrrolidone are added to purified water, dissolved by heating to 50-65°C.
*Step B*: kaolin is dispersed in a D-sorbitol solution.
*Step C*: titanium dioxide is dispersed in purified water.
*Step D:* EDTA is dissolved in purified water.
*Step E*: methylparaben and propylparaben are dissolved in propylene glycol.
*Step F*: tartaric acid is dissolved in water.
*Step G*: CMC sodium, sodium polyacrylate and aluminium glycinate are dispersed in butylene glycol.
*Step H*: propylene glycol is added to water in a ratio of 1:1 parts by weight and the entire quantity of diclofenac salt with a cyclic organic base chosen from hydroxyethylpyrrolidine or hydroxyethylpiperidine is dissolved in the mixture thus obtained, heating to 30-50°C.

3. Process according to claim 2, **characterized by** mixing together the solutions or dispersions obtained in said steps from A to H, mixing them in the following order:
- product of Step A
- D-sorbitol in solution
- product of Step B
- purified water
- product of Step C
- product of Step D
- product of Step E after adding polysorbate 80
- said product of Step F
- add said product of Step G and mix
- add said product of Step H and mix until the mixture is homogeneous, thus giving rise to said composition.

4. Process according to claim 3, **characterized by** mixing together the solutions or dispersions obtained in said steps from A to H, mixing them in the following order:
- product of Step A
- D-sorbitol in solution
- product of Step B
- purified water
- product of Step C
- product of Step D and a rinse with purified water
- product of Step E after adding polysorbate 80
- fragrance
- said product of Step F
- add said product of Step G and mix
- add said product of Step H, then rinsing with purified water and mixing until the mixture is homogeneous, thus giving rise to said composition.

## Patentansprüche

1. Verfahren zur Herstellung einer pharmazeutischen Zusammensetzung mit entzündungshemmender und schmerzlindernder Aktivität zur Verabreichung mittels eines Pflasters zur äußerlichen Anwendung, wobei ihre Bestandteile als Wirkprinzip ein Salz von Diclofenac, 2[(2,6-Dichlorphenyl)amino]benzol-Essigsäure, mit einer zyklischen organischen Base, die aus Hydroxyethylpyrrolidin oder Hydroxyethylpiperidin ausgewählt ist, umfassen, **dadurch gekennzeichnet, dass** das Wirkprinzip zu einer Mischung einer oder mehrerer dieser Verbindungen in Form einer Lösung in zwei Lösungsmitteln, und zwar Wasser und Propylenglycol, in einem Verhältnis von 1:1 Gewichtsteilen zugegeben wird.

2. Verfahren nach Anspruch 1, **dadurch gekennzeichnet, dass** Lösungen oder Dispersionen der Bestandteile wie in den folgenden Schritten beschrieben getrennt hergestellt werden:
*Schritt A*: Gelatine und Polyvinylpyrrolidon werden zu aufgereinigtem Wasser zugegeben, durch Erhitzen auf 50-65°C aufgelöst.
*Schritt B*: Kaolin wird in einer D-Sorbitollösung dispergiert.
*Schritt C*: Titandioxid wird in aufgereinigtem Wasser dispergiert.
*Schritt D*: EDTA wird in aufgereinigtem Wasser aufgelöst.
*Schritt E*: Methylparaben und Propylparaben werden in Propylenglycol aufgelöst.
*Schritt F*: Weinsäure wird in Wasser aufgelöst.
*Schritt G*: CMC-Natrium, Natriumpolyacrylat und Aluminiumglycinat werden in Butylenglycol dispergiert.
*Schritt H*: Propylenglycol wird in einem Verhältnis von 1:1 Gewichtsteilen in Wasser gegeben und die vollständige Menge an dem Salz von Diclofenac wird mit einer zyklischen organischen Base, die aus Hydroxyethylpyrrolidin oder Hydroxyethylpiperidin ausgewählt ist, in der so erhaltenen Mischung aufgelöst, Erhitzen auf 30-50°C.

3. Verfahren nach Anspruch 2, **dadurch gekennzeichnet, dass** die in den Schritten A bis H erhaltenen Lösungen oder Dispersionen zusammen gemischt werden, wobei sie in der folgenden Reihenfolge gemischt werden:
- Erzeugnis von Schritt A
- D-Sorbitol in Lösung
- Erzeugnis von Schritt B
- aufgereinigtes Wasser
- Erzeugnis von Schritt C
- Erzeugnis von Schritt D
- Erzeugnis von Schritt E nach der Zugabe von Polysorbat 80
- das Erzeugnis von Schritt F
- Zugeben des Erzeugnisses von Schritt G und Mischen
- Zugeben des Erzeugnisses von Schritt H und Mischen bis die Mischung homogen ist, wodurch sich die Zusammensetzung bildet.

4. Verfahren nach Anspruch 3, **dadurch gekennzeichnet, dass** die in Schritt A bis H erhaltenen Lösungen oder Dispersionen zusammen gemischt werden, wobei sie in der folgenden Reihenfolge gemischt werden:
- Erzeugnis von Schritt A
- D-Sorbitol in Lösung
- Erzeugnis von Schritt B
- aufgereinigtes Wasser
- Erzeugnis von Schritt C
- Erzeugnis von Schritt D und ein Spülschritt mit aufgereinigtem Wasser
- Erzeugnis von Schritt E nach der Zugabe von Polysorbat 80
- Duftstoff
- das Erzeugnis von Schritt F
- Zugeben des Erzeugnisses von Schritt G und Mischen
- Zugeben des Erzeugnisses von Schritt H und anschließend Spülen mit aufgereinigtem Wasser und Mischen bis die Mischung homogen ist, wodurch sich die Zusammensetzung bildet.

## Revendications

1. Procédé pour préparer une composition pharmaceutique ayant une activité anti-inflammatoire et analgésique pour administration via un timbre à usage externe, ses composants contenant, en tant que principe actif, un sel de diclofénac, l'acide 2-[(2,6-dichlorophényl)amino]benzène-acétique, avec une base organique cyclique choisie parmi l'hydroxyéthyl pyrrolidine et l'hydroxyéthyl pipéridine, **caractérisé en ce que** ledit principe actif est ajouté à un mélange d'un ou plusieurs desdits composants, sous la forme d'une solution dans deux solvants, à savoir l'eau et le propylène glycol, en un rapport de 1/1 partie en poids.

2. Procédé selon la revendication 2, **caractérisé par** la préparation séparée de solutions ou dispersions desdits composants comme décrit dans les étapes suivantes :
*étape A* : de la gélatine et de la polyvinyl pyrrolidone sont ajoutées à de l'eau purifiée et dissoutes par chauffage à 50-65°C,
*étape B* : du kaolin est dispersé dans une solution de D-sorbitol,
*étape C* : du dioxyde de titane est dispersé dans de l'eau purifiée,
*étape D* : de l'EDTA est dissous dans de l'eau purifiée,
*étape E :* du méthyl parabène et du propyl parabène sont dissous dans du propylène glycol,
*étape F* : de l'acide tartrique est dissous dans de l'eau,
*étape G* : de la CMC de sodium, du polyacrylate de sodium et du glycinate d'aluminium sont dispersés dans du butylène glycol,
*étape H* : du propylène glycol est ajouté à de l'eau en un rapport de 1/1 partie en poids,
et la quantité totale de sel de diclofénac avec une base organique cyclique choisie parmi l'hydroxyéthyl pyrrolidine et l'hydroxyéthyl pipéridine est dissoute dans le mélange ainsi obtenu, avec chauffage à 30-50°C.

3. Procédé selon la revendication 2, **caractérisé en ce que** les solutions ou dispersions obtenues dans lesdites étapes A à H sont mélangées ensemble, en étant mélangées dans l'ordre suivant :
- produit de l'étape A,
- D-sorbitol en solution,
- produit de l'étape B,
- eau purifiée,
- produit de l'étape C,
- produit de l'étape D,
- produit de l'étape E après addition de polysorbate 80,
- ledit produit de l'étape F,
- addition dudit produit de l'étape G et mélange,
- addition dudit produit de l'étape H et mélange jusqu'à ce que le mélange soit homogène, ce qui donne ainsi ladite composition.

4. Procédé selon la revendication 3,
**caractérisé en ce que** les solutions ou dispersions obtenues dans lesdites étapes A à H sont mélangées ensemble, en étant mélangées dans l'ordre suivant :
- produit de l'étape A,
- D-sorbitol en solution,
- produit de l'étape B,
- eau purifiée,
- produit de l'étape C,
- produit de l'étape D et rinçage à l'eau purifiée,
- produit de l'étape E après addition de polysorbate 80,
- parfum,
- ledit produit de l'étape F,
- addition dudit produit de l'étape G et mélange,
- addition dudit produit de l'étape H puis rinçage à l'eau purifiée et mélange jusqu'à ce que le mélange soit homogène, ce qui donne ainsi ladite composition.
